# EUROPÄISCHE PATENTSCHRIFT

(11) **EP 1 270 543 B1**
(45) Veröffentlichungstag und Bekanntmachung des Hinweises auf die Patenterteilung: **07.10.2009**
(21) Anmeldenummer: 02013584.4
(22) Anmeldetag: 20.06.2002
(51) Int. Cl.: C07C 209/64, C07C 211/13

(54) **Verfahren zur Herstellung von sekundären Aminen aus primären Aminen**
Process for the preparation of primary amines from secondary amines
Procédé pour préparer des amines secondaires à partir d'amines primaires

(30) Priorität: 21.06.2001 DE 10129908
(43) Veröffentlichungstag der Anmeldung: 02.01.2003
(73) Patentinhaber: BASF SE, 67056 Ludwigshafen (DE)
(72) Erfinder: Neumann, Peter, Dr., 68309 Mannheim (DE); Melder, Johann-Peter, Dr., 67459 Böhl-Iggelheim (DE); Benisch, Christoph, Dr., 69214 Eppelheim (DE); Höhn, Arthur, Dr., 67281 Kirchheim (DE); Pfeffinger, Joachim, 67063 Ludwigshafen (DE)
(74) Vertreter: Isenbruck, Günter

(56) Entgegenhaltungen:
- DE-A- 3 048 832

## Beschreibung

Die vorliegende Erfindung betrifft ein Verfahren zur Herstellung von sekundären Aminen aus primären Aminen an Übergangsmetall-Katalysatoren.

Verfahren zur Herstellung von sekundären Aminen aus primären Aminen sind an sich bekannt. Es ist dabei Stand der Technik, ein primäres Amin, das die gewünschten Substituenten bzw. Strukturelemente aufweist, bei den jeweils gewählten Reaktionsbedingungen unter Wasserstoff zu dem gewünschten sekundären Amin umzusetzen. Es werden verschiedenste Katalysatoren eingesetzt; die bei der Reaktion angewandten Drücke und Temperaturen variieren stark. Ein Hauptproblem bei der beschriebenen Synthese der sekundären Amine liegt in dem Umsatz bzw. der Selektivität zu dem gewünschten Produkt, die häufig nicht die gewünschten Werte erreichen. Oft erweist sich auch die Verwendung teurer edelmetallhaltiger Katalysatoren als notwendig.

Die DE-A 30 48 832 betrifft ein Verfahren zur Herstellung von Aminen, insbesondere Bis(3-dimethylamino)propylamin (Bis-DMAPA) aus 3-Dimethylaminopropionitril (DMAPN) oder 3-Dimethylaminopropylamin (DMAPA) bzw. Gemischen von DMAPN und DMAPA. In einem Beispiel wird bei hohem Druck (173 bar) an Ni-Cu-Cr₂O₃ bei einem DMAPA-Umsatz von 53% eine Bis-DMAPA-Selektivität von 80% erreicht, an Co-Cu-Cr₂O₃ bei einem DMAPA-Umsatz von 49% eine Bis-DMAPA-Selektivität von 88%.

Es zeigt sich hier, dass insbesondere die Umsätze bei deutlich zu niedrigen Werten liegen. Auch wird aufgrund von Umweltaspekten die Verwendung chromhaltiger Katalysatoren heutzutage nicht mehr akzeptiert.

Die Aufgabe der vorliegenden Erfindung besteht darin, ein Verfahren zur Herstellung sekundärer Amine aus primären Aminen zur Verfügung zu stellen, das mit chromfreien Katalysatoren betrieben werden kann und die gewünschten sekundären Amine in hohen Ausbeuten und Selektivitäten liefert.

Diese Aufgabe wird gelöst durch ein Verfahren zur Herstellung eines sekundären Amins der allgemeinen Formel 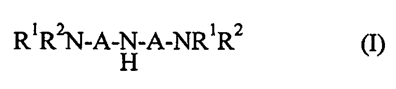 in der
- R¹R²: gleich oder verschieden sein können und unabhängig voneinander einen linearen oder verzweigten Alkylrest mit 1 bis 20 C-Atomen, der mit 1 bis 5 Phenylgruppen substituiert sein kann, oder einen Cyclohexylrest darstellen oder zusammen mit dem N-Atom an das sie gebunden sind, einen 3- bis 7-gliedrigen gesättigten Ring bilden, der gegebenenfalls weitere Heteroatome aus der Gruppen N, O und S enthalten und mit 1 bis 5 Alkylgruppen mit 1 oder 2 C-Atomen substituiert sein kann.
- A: eine lineare oder verzweigte Alkylengruppe mit 2 bis 20 C-Atomen darstellt, die in ihrer Kette gegebenenfalls 1 bis 5 Phenylengruppen enthalten kann, oder einen Rest der Formel 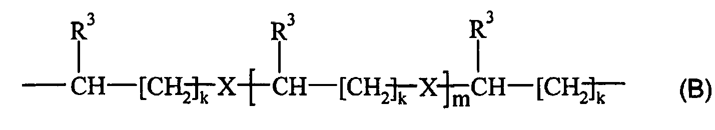
darstellt, in der R³ = H oder CH₃, X = O oder S oder eine NR⁴-Gruppe, in der R⁴ H oder eine lineare oder verzweigte Alkylgruppe mit 1 bis 4 C-Atomen darstellt, k eine ganze Zahl von 1 bis 2 ist und m eine ganze Zahl von 0 bis 4 ist, oder eine Gruppe der Formel 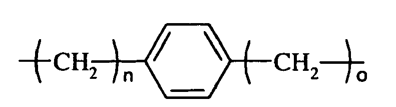 oder 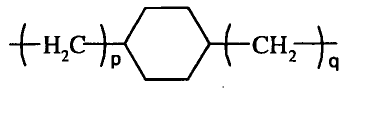 in der n, o bzw. p, q jeweils unabhängig voneinander ganze Zahlen von 1 bis 4 darstellen,
durch Umsetzung von primären Aminen der allgemeinen Formel

R¹R²N-A-NH₂ (II),

in der R¹, R² und A die bei Formel (I) gegebene Bedeutung haben, in Anwesenheit von Wasserstoff sowie eines chromfreien Katalysators, der mindestens ein Element oder eine Verbindung eines Elements aus der Gruppe Nickel, Kobalt und Kupfer enthält. Die Katalysatormasse kann in einer Ausführungsform der vorliegenden Erfindung kein Metall oder eine Verbindung davon aus der Gruppe IB enthalten. In einer bevorzugten Ausführungsform enthält der Katalysator bis zu 50 Gew.-% mindestens eines Elements oder einer Verbindung eines Elements aus der Gruppe IB des Periodensystems der Elemente.

Die Katalysatoren, die in dem erfindungsgemäßen Verfahren eingesetzt werden, enthalten in der aktiven Katalysatormasse also bis zu 100 Gew.-% mindestens ein Element oder mindestens eine Verbindung eines Elements aus der Gruppe Nickel, Kobalt und Kupfer. Die aktive Katalysatormasse kann in einer Ausführungsform 0 Gew.-%, in einer bevorzugten Ausführungsform bis zu 50 Gew.-%, mindestens eines Elements oder mindestens einer Verbindung eines Elements aus der Gruppe IB des Periodensystems der Elemente enthalten, also aus der Gruppe bestehend aus Cu, Ag und Au, vorzugsweise Cu. Die Menge an Metall oder Verbindung eines Metalls der Gruppe IB liegt in einer weiter bevorzugten Ausführungsform bei 1 bis 30 Gew.-%, insbesondere bei 10 bis 25 Gew.-% bezogen auf die Gesamtmenge der aktiven Katalysatormasse. In einer weiteren bevorzugten Ausführungsform enthält die aktive Katalysatormasse mindestens ein Element oder mindestens eine Verbindung davon aus der Gruppe bestehend aus Ni, Co, Cu, Ru, Rh, Ir, Pd, Pt, in den vorstehend angegebenen Mengenverhältnissen für die allgemeinen und bevorzugten Ausführungsformen.

Werden zur Herstellung des Katalysators Verbindungen der genannten Metalle eingesetzt, können beispielsweise die Oxide, Nitrate, Carbonate, Chloride und Acetate verwendet werden.

In der meist bevorzugten Ausführungsform der vorliegenden Erfindung werden bei der Herstellung des Katalysators die Oxide der verwendeten Elemente eingesetzt. Diese werden dann vor dem Einsatz in die Reaktion reduziert, vorzugsweise durch Behandeln mit Wasserstoff. So entsteht ein Katalysator, der die anwesenden Metallkomponenten in elementarer, feinverteilter Form enthält.

Die Katalysatoren können als Vollkontakt oder in geträgerter Form eingesetzt werden. Bei Einsatz von geträgerten Katalysatoren beträgt der Anteil des Trägers an der Gesamtmasse des Katalysators (Aktivmasse plus Träger) 10 bis 90 Gew.-%.

Als Träger können alle bekannten geeigneten Träger verwendet werden, beispielsweise Aktivkohle, Siliciumcarbid oder Metalloxide. Der Einsatz von Metalloxiden ist bevorzugt. Von den Metalloxiden werden vorzugsweise Aluminiumoxid, Siliciumdioxid, Titandioxid, Zirkoniumdioxid, Zinkoxid, Magnesiumoxid oder deren Gemische verwendet, die gegebenenfalls mit Alkali- und/oder Erdalkalimetalloxiden dotiert sind. Besonders bevorzugt sind γ-Aluminiumoxid, Siliciumdioxid, Zirkoniumdioxid oder Titandioxid oder Gemische davon, insbesondere Al₂O₃. Die Träger können in beliebiger Form eingesetzt werden, beispielsweise als Extrudate (in Form von Strängen), Pellets, Tabletten, Monolithe, Gewebe, Gestricke oder pulverförmig. Die geträgerten Katalysatoren können nach allgemein bekannten Verfahren hergestellt werden. Hierzu zählt etwa das Tränken eines Trägers mit Lösungen von Verbindungen der eingesetzten Metallkomponenten. Als Lösungsmittel eignen sich alle üblichen Lösungsmittel, etwa Wasser, Methanol, Ethanol oder Aceton, vorzugsweise wird Wasser eingesetzt. Weiterhin kann der Katalysator durch gemeinsames oder sequentielles Fällen der Katalysatorkomponenten, anschließende Filtration und Waschen des Filterkuchens hergestellt werden. An das Tränken bzw. Fällen schließen sich ein Trocknungsschritt (50 bis 200°C) und ein Calcinationsschritt (200 bis 500°C) an. Die Katalysatoren werden dann reduziert bei Endtemperaturen von 200 bis 400°C und können anschließend passiviert werden, da die reduzierten Metalle pyrophor sind. Nach Einbau der Katalysatoren in den Synthesereaktor können die Katalysatoren vor Anfahren der Reaktion durch Reduktion mit Wasserstoff bei Temperaturen zwischen 100 und 300°C reaktiviert werden.

Erfindungsgemäß ist der Einsatz von primären Aminen der Formel (I) bzw. die Synthese von sekundären Aminen der Formel (II) bevorzugt, in denen die Substituenten R¹, R² und A die folgende Bedeutung haben:
- A: ist eine lineare oder verzweigte Methylenkette mit 2 bis 10 C-Atomen oder eine Gruppe der Formel

- CH₂-CH₂-O-(CH₂-CH₂-O)ₙ-CH₂-CH₂ -,

in der n eine ganze Zahl von 0 bis 2 ist.
- R¹, R²: sind gleich oder verschieden und stehen unabhängig voneinander für einen Alkylrest mit 1 bis 12 C-Atomen oder bilden zusammen mit dem Stickstoffatom, an das sie gebunden sind, einen 5- oder 6-gliedrigen gesättigten Ring, der gegebenenfalls ein weiteres Heteroatom aus der Gruppe O und N enthalten kann.

Besonders bevorzugt sind Amine der Formel (I) und (II), in denen
- A: eine Alkylengruppe mit 1 bis 6 C-Atomen oder eine Gruppe der Formel

- CH₂-CH₂-O-CH₂-CH₂ -

ist und
R¹ und R² gleich oder verschieden sein können und unabhängig voneinander einen linearen oder verzweigten Alkylrest mit 1 bis 4 Kohlenstoffatomen darstellen oder gemeinsam mit dem Stickstoffatom, an das sie gebunden sind, einen Piperidinring oder einen Morpholinring bilden.

Insbesondere wird 3-Dimethylaminopropylamin (DMAPA) eingesetzt, wobei Bis-3-Dimethylaminopropylamin (Bis-DMAPA) gebildet wird.

Das erfindungsgemäße Verfahren wird bei Temperaturen von 50 bis 250°C, vorzugsweise bei 90°C bis 170°C, besonders bevorzugt bei 120°C bis 160°C und Drücken von 5 bis 350 bar, vorzugsweise 5 bis 200 bar, besonders bevorzugt 10 bis 100 bar, insbesondere 10 bis 30 bar, diskontinuierlich oder bevorzugt kontinuierlich in Druckapparaturen wie Autoklaven oder bevorzugt in einem Rohrreaktor durchgeführt. Der Druck ist dabei vorzugsweise der Wasserstoffdruck im Reaktor. Bei Verwendung eines Rohrreaktors kann der verwendete Katalysator auch als Festbettkatalysator vorliegen.

Die Umsetzung kann in der Gasphase oder in Flüssigphase erfolgen.

Die Katalysatorbelastung, bezogen auf das eingesetzte primäre Amin, beträgt vorzugsweise 0,1 bis 2 kg 1⁻¹h⁻¹, insbesondere 0,8 bis 1,2 kg 1⁻¹h⁻¹. Dabei kann ein Teil des flüssigen Reaktionsaustrags in die Umsetzung zurückgeführt werden.

Das erfindungsgemäße Verfahren lässt sich lösungsmittelfrei oder in Lösungsmitteln wie Wasser, Methanol, Ethanol, Tetrahydrofuran, Methyl-tert-butylether oder N-Methylpyrrolidon durchführen. Im Lösungsmittel kann dabei das eingesetzte primäre Amin gelöst sein. Das Lösungsmittel kann auch getrennt dem Reaktor an beliebiger Stelle zugeführt werden. Vorzugsweise wird lösungsmittelfrei gearbeitet.

Das mit dem erfindungsgemäßen Verfahren erhaltene gewünschte sekundäre Amin lässt sich in an sich bekannter Weise, beispielsweise destillativ, vom Reaktionsgemisch abtrennen und reinigen.

Beispielsweise ist es möglich, durch Rektifikation einen Strom mit reinem sekundärem Amin und einen Strom mit primärem Amin zu erhalten, wobei der Strom mit dem primären Amin in die Synthese zurückgeführt wird.

Erfindungsgemäß werden vorzugsweise die primären Amine der allgemeinen Formel (II) und die sekundären Amine der allgemeinen Formel (I) in einem Gewichtsverhältnis von 10:1 bis 1:10 erhalten, vorzugsweise 2 :3-4.

Das erfindungsgemäße Verfahren gestattet es, Endproduktgemische zu erhalten, die nur geringe Mengen an tertiären Aminen enthalten, generell in Mengen < 10 Gew.-%. Es ist auch möglich, das Verfahren so durchzuführen, dass < 5 Gew.-% an tertiären Aminen erhalten werden. Bei optimaler Reaktionsführung ist es auch möglich, dass keine tertiären Amine gebildet werden.

Die mit dem erfindungsgemäßen Verfahren erhältlichen Amine, vorzugsweise Bis-DMAPA, sind Härter für Epoxyharze, Katalysatoren für Polyurethane, Zwischenprodukte zur Herstellung quaternärer Ammoniumverbindungen, Weichmacher, Korrosionsinhibitoren, Textilhilfsmittel, Farbstoffe und Emulgatoren. Mehrfach funktionalisierte tertiäre Amine dienen außerdem zur Herstellung von Kunstharzen, Ionenaustauschern, Pharmazeutika, Pflanzenschutz- und Schädlingsbekämpfungsmitteln.

### Die Erfindung wird nun in dem nachstehenden Beispiel näher erläutert:

### Beispiel

Ein beheizter Rohrreaktor mit 10 mm Innendurchmesser, einem zentral angebrachten Thermoelement und einer Gesamtlänge von 35 cm wird mit 89 g des Katalysators gefüllt. Der Katalysator besteht aus 42 Gew.-% CoO, 42 Gew.-% NiO und 16 Gew.-% CuO auf einem Träger aus Aluminiumoxid (76 Gew.-% der Katalysatorgesamtmasse).

Vor der Reaktion wurde der Katalysator bei 180°C aktiviert, zunächst in einem Gasstrom von Stickstoff und Wasserstoff 4:1, anschließend in einem Gasstrom von Stickstoff und Wasserstoff 1:1 und schließlich in reinem Wasserstoff.

Durch den Reaktor wurden von unten nach oben 1200g/(1*h) an 3-Dimethylaminopropylamin (DMAPA) und 20Nl/h Wasserstoff zugegeben. Der Reaktor wird bei einer Temperatur von 140°C und einem Gesamtdruck von 30 bar gehalten.

Das aus dem Reaktor austretende Gemisch wurde abgekühlt und auf Normaldruck entspannt. Mittels gaschromatographischer Analyse wurde im Reaktoraustrag ein Gehalt von 52 Gew.-% Bis-(3-dimethylaminopropyl)amin (Bis-DMAPA), 42 Gew.-% an 3-Dimethylaminopropylamin (DMAPA) und 6 Gew.-% an verschiedenen Nebenprodukten gefunden. Der DMAPA-Umsatz lag damit bei 58%, die Selektivität zu Bis-DMAPA bei 90%.

In einer Labordestillation mit Füllkörperschüttung (10 theoretische Trennstufen) wurden anschließend 300 g des flüssigen Reaktoraustrags unter vermindertem Druck und einem Rücklaufverhältnis von 5:1 diskontinuierlich aufdestilliert. Es wurde eine Fraktion mit 157g mit einem Gehalt von 97,4 Gew.-% an DMAPA und eine zweite Fraktion mit 129 g mit einem Gehalt von 98,4 Gew.-% an Bis-DMAPA erhalten.

## Patentansprüche

1. Verfahren zur Herstellung eines sekundären Amins der allgemeinen Formel 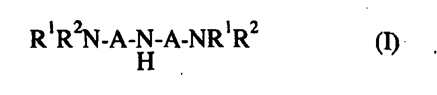 in der
R¹R² gleich oder verschieden sein können und unabhängig voneinander einen linearen oder verzweigten Alkylrest mit 1 bis 20 C-Atomen, der mit 1 bis 5 Phenylgruppen substituiert sein kann, oder einen Cyclohexylrest darstellen oder zusammen mit dem N-Atom an das sie gebunden sind, einen 3- bis 7-gliedrigen gesättigten Ring bilden, der gegebenenfalls weitere Heteroatome aus der Gruppen N, O und S enthalten und mit 1 bis 5 Alkylgruppen mit 1 oder 2 C-Atomen substituiert sein kann
A eine lineare oder verzweigte Alkylengruppe mit 2 bis 20 C-Atomen darstellt, die in ihrer Kette gegebenenfalls 1 bis 5 Phenylengruppen enthalten kann, oder einen Rest der Formel 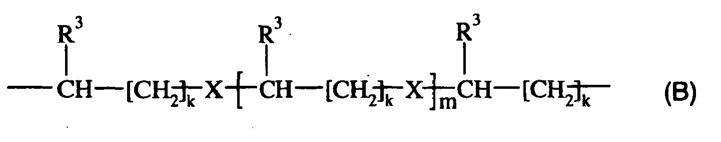 darstellt , in der R³ = H oder CH₃, X = O oder S oder eine NR⁴-Gruppe, in der R⁴ H oder eine lineare oder verzweigte Alkylgruppe mit 1 bis 4 C-Atomen darstellt, k eine ganze Zahl von 1 bis 2 ist und m eine ganze Zahl von 0 bis 4 ist, oder eine Gruppe der Formel 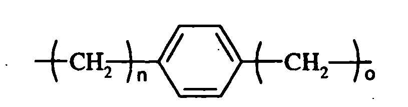 oder 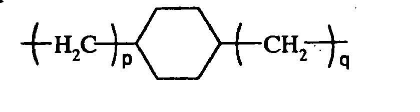 in der n, o bzw. p, q jeweils unabhängig voneinander ganze Zahlen von 1 bis 4 darstellen,
durch Umsetzung von primären Aminen der allgemeinen Formel
R¹R²N-A-NH₂ (II),
in der R¹, R² und A die bei Formel (I) gegebene Bedeutung haben, in Anwesenheit von Wasserstoff sowie eines chromfreien Katalysators, der mindestens ein Element oder eine Verbindung eines Elementes aus der Gruppe Nickel, Kobalt und Kupfer enthält.

2. Verfahren nach Anspruch 1, **dadurch gekennzeichnet, dass** in den Aminen (I) und (II) die Substituenten R¹, R² und A die folgende Bedeutung haben:
A ist eine lineare oder verzweigte Methylenkette mit 2 bis 10 C-Atomen oder eine Gruppe der Formel
- CH₂-CH₂-O-(CH₂-CH₂-O)n-CH₂-CH₂ -,
in der n eine ganze Zahl von 0 bis 2 ist.
R¹, R² sind gleich oder verschieden und stehen unabhängig voneinander für einen Alkylrest mit 1 bis 12 C-Atomen oder bilden zusammen mit dem Stickstoffatom, an das sie gebunden sind, einen 5- oder 6-gliedrigen gesättigten Ring, der gegebenenfalls ein weiteres Heteroatom aus der Gruppe O und N enthalten kann.

3. Verfahren nach Anspruch 2, **dadurch gekennzeichnet, dass** in den Aminen (I) und (II)
A eine Alkylengruppe mit 1 bis 6 C-Atomen oder eine Gruppe der Formel
- CH₂-CH₂-O-CH₂-CH₂ -
ist und
R¹ und R² gleich oder verschieden sein können und unabhängig voneinander einen linearen oder verzweigten Alkylrest mit 1 bis 4 Kohlenstoffatomen darstellen oder gemeinsam mit dem Stickstoffatom, an das sie gebunden sind, einen Piperidinring oder einen Morpholinring bilden.

4. Verfahren nach Anspruch 3, **dadurch gekennzeichnet, dass** 3-Dimethylaminopropylamin zu Bis-3-Dimethylaminopropylamin umgesetzt wird.

5. Verfahren nach einem der Ansprüche 1 bis 4, **dadurch gekennzeichnet, dass** der Katalysator in der Aktivmasse 0 Gew.-% Kupfer oder einer Kupfer-Verbindung aufweist, vorzugsweise bis zu 50 Gew.-%, mehr bevorzugt 1 bis 30 Gew.-%, insbesondere 10 bis 25 Gew.-%, bezogen auf die Gesamtmenge der aktiven Katalysatormasse.

6. Verfahren nach einem der Ansprüche 1 bis 5, **dadurch gekennzeichnet, dass** der Katalysator auf einen geeigneten Träger angebracht ist, vorzugsweise einem Träger aus Aktivkohle, Siliciumcarbid, Metalloxiden oder Gemischen davon, mehr bevorzugt Aluminiumoxid, Siliciumdioxid, Titandioxid, Zirkoniumdioxid, Zinkoxid, Magnesiumoxid oder Gemischen davon, insbesondere γ-Aluminiumoxid, Siliciumdioxid, Zirkoniumdioxid, Titandioxid oder Gemischen davon, und wobei der Träger vorzugsweise 10 bis 90 Gew.-% der Gesamtmasse des Katalysators ausmacht.

7. Verfahren nach einem der Ansprüche 1 bis 6, **dadurch gekennzeichnet, dass** das Verfahren bei Drücken von 5 bis 350 bar, vorzugsweise 5 bis 200 bar, besonders bevorzugt 10 bis 100 bar, insbesondere 10 bis 30 bar, und Temperaturen von 50 bis 250°C, vorzugsweise 90 bis 170°C, insbesondere 120 bis 160°C durchgeführt wird.

8. Verfahren nach einem der Ansprüche 1 bis 7, **dadurch gekennzeichnet, dass** das Verfahren bei einer Katalysatorbelastung von 0,1 bis 2 kg l⁻¹ h⁻¹, vorzugsweise 0,8 bis 1,2 kg l⁻¹ h⁻¹ bezogen auf das primäre Amin durchgeführt wird.

9. Verfahren nach einem der Ansprüche 1 bis 8, **dadurch gekennzeichnet, dass** das Verfahren diskontinuierlich oder kontinuierlich, vorzugsweise kontinuierlich, insbesondere in einem Rohrreaktor, durchgeführt wird.

## Claims

1. A process for preparing a secondary amine of the formula 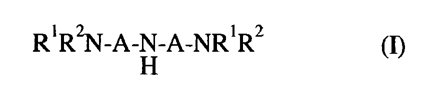 where
R¹, R² may be identical or different and are each, independently of one another, a linear or branched alkyl radical having from 1 to 20 carbon atoms which may bear from 1 to 5 phenyl groups as substituents or a cyclohexyl radical or together with the N atom to which they are bound form a 3- to 7-membered saturated ring which may comprise further heteroatoms selected from the group consisting of N, O and S and may be substituted by from 1 to 5 alkyl groups having 1 or 2 carbon atoms,
A is a linear or branched alkylene group having from 2 to 20 carbon atoms which may comprise from 1 to 5 phenylene groups in its chain, or a radical of the formula 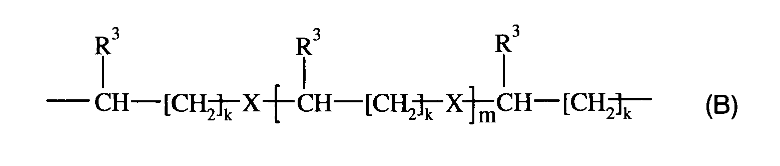
where R³ = H or CH₃ , X = O or S or an NR⁴ group in which R⁴ is H or a linear or branched alkyl group having from 1 to 4 carbon atoms, k is 1 or 2 and m is an integer from 0 to 4, or a group of the formula 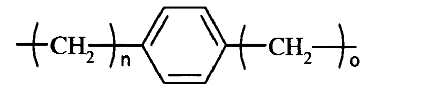 or 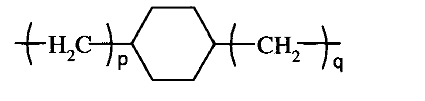 where n, o and p, q are each, independently of one another, integers from 1 to 4,
by reaction of primary amines of the formula
R¹R²N-A-NH₂ (II),
where R¹, R² and A are as defined for formula (I), in the presence of hydrogen and a chromium-free catalyst comprising at least one element or compound of an element of the group nickel, cobalt and copper.

2. The process according to claim 1, wherein the substituents R¹, R² and A in the amines (I) and (II) have the following meanings:
A is a linear or branched methylene chain having from 2 to 10 carbon atoms or a group of the formula
-CH₂-CH₂-O- (CH₂-CH₂-O)ₙ-CH₂-CH₂-,
where n is an integer from 0 to 2,
R¹, R² are identical or different and are each, independently of one another, an alkyl radical having from 1 to 12 carbon atoms or together with the nitrogen atom to which they are bound form a 5- or 6-membered saturated ring which may comprise a further heteroatom selected from the group consisting of O and N.

3. The process according to claim 2, wherein, in the amines (I) and (II),
A is an alkylene group having from 1 to 6 carbon atoms or a group of the formula
-CH₂-CH₂-O-CH₂-CH₂-
and
R¹ and R² may be identical or different and are each, independently of one another, a linear or branched alkyl radical having from 1 to 4 carbon atoms or together with the nitrogen atom to which they are bound form a piperidine ring or a morpholine ring.

4. The process according to claim 3, wherein 3-dimethylaminopropylamine is converted into bis-3-dimethylaminopropylamine.

5. The process according to any of claims 1 to 4, wherein the catalyst comprises, in the active composition, 0% by weight of copper or a copper compound, preferably up to 50% by weight, more preferably from 1 to 30% by weight, in particular from 10 to 25% by weight, based on the total amount of active catalyst composition.

6. The process according to any of claims 1 to 5, wherein the catalyst has been applied to a suitable support, preferably a support comprising activated carbon, silicon carbide, metal oxides or mixtures thereof, more preferably aluminum oxide, silicon dioxide, titanium dioxide, zirconium dioxide, zinc oxide, magnesium oxide or mixtures thereof, in particular γ-aluminum oxide, silicon dioxide, zirconium dioxide, titanium dioxide or mixtures thereof, and the support preferably makes up from 10 to 90% by weight of the total mass of the catalyst.

7. The process according to any of claims 1 to 6 carried out at from 50 to 250°C, preferably from 90 to 170°C, in particular from 120 to 160°C, and pressures of from 5 to 350 bar, preferably from 5 to 200 bar, particularly preferably from 10 to 100 bar, in particular from 10 to 30 bar.

8. The process according to any of claims 1 to 7 carried out at a space velocity over the catalyst of from 0.1 to 2 kg l⁻¹ h⁻¹, preferably from 0.8 to 1.2 kg l⁻¹ h⁻¹, based on the primary amine.

9. The process according to any of claims 1 to 8 carried out batchwise or continuously, preferably continuously, in particular in a tube reactor.

## Revendications

1. Procédé de fabrication d'une amine secondaire de formule générale 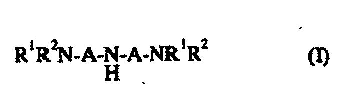 dans laquelle
R¹R² peuvent être identiques ou différents et représentent indépendamment l'un de l'autre un radical alkyle linéaire ou ramifié contenant 1 à 20 atomes C, qui peut être substitué avec 1 à 5 groupes phényle, ou un radical cyclohexyle, ou forment ensemble avec l'atome N auquel ils sont reliés un cycle saturé à 3 à 7 éléments qui peut éventuellement contenir des hétéroatomes supplémentaires du groupe N, O, S et être substitué avec 1 à 5 groupes alkyle contenant 1 ou 2 atomes C
A représente un groupe alkylène linéaire ou ramifié contenant 2 à 20 atomes C, qui peut éventuellement contenir dans sa chaîne 1 à 5 groupes phénylène, ou un radical de formule 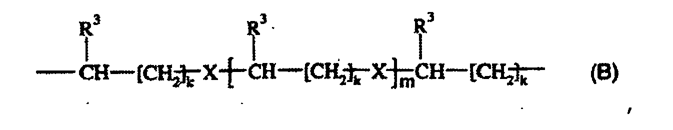
dans laquelle R³ = H ou CH₃ , X = O ou S ou un groupe NR⁴, dans lequel R⁴ représente H ou un groupe alkyle linéaire ou ramifié contenant 1 à 4 atomes C, k est un nombre entier de 1 à 2 et m est un nombre entier de 0 à 4, ou un groupe de formule 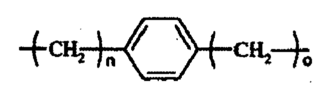 ou 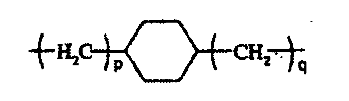 dans lesquelles n, o ou p, q représentent à chaque fois indépendamment l'un de l'autre des nombres entiers de 1 à 4,
par réaction d'amines primaires de formule générale
**R¹R²N-A-NH₂** **(II),**
dans laquelle R¹, R² et A ont la signification donnée pour la formule (I), en présence d'hydrogène et d'un catalyseur sans chrome, qui contient au moins un élément ou un composé d'un élément du groupe du nickel, du cobalt et du cuivre.

2. Procédé selon la revendication 1, **caractérisé en ce que** dans les amines (I) et (II), les substituants R¹, R² et A ont la signification suivants :
A est une chaîne méthylène linéaire ou ramifiée contenant 2 à 10 atomes C ou un groupe de formule
**-CH₂-CH₂O-(CH₂-CH₂-O)ₙ-CH₂-CH₂-**
dans laquelle n est un nombre entier de 0 à 2,
R¹, R² sont identiques ou différents et représentent indépendamment l'un de l'autre un radical alkyle contenant 1 à 12 atomes C ou forment ensemble avec l'atome d'azote auquel ils sont reliés un cycle saturé à 5 ou 6 éléments qui peut éventuellement contenir un hétéroatome supplémentaire du groupe O et N.

3. Procédé selon la revendication 2, **caractérisé en ce que** dans les amines (I) et (II)
A est un groupe alkylène contenant 1 à 6 atomes C ou un groupe de formule
**-CH₂-CH₂-O-CH₂-CH₂-**
et
R¹ et R² peuvent être identiques ou différents et représentent indépendamment l'un de l'autre un radical alkyle linéaire ou ramifié contenant 1 à 4 atomes de carbone ou forment ensemble avec l'atome d'azote auquel ils sont reliés un cycle pipéridine ou un cycle morpholine.

4. Procédé selon la revendication 3, **caractérisé en ce que** de la 3-diméthylaminopropylamine est transformée en bis-3-diméthylaminopropylamine.

5. Procédé selon l'une quelconque des revendications 1 à 4, **caractérisé en ce que** le catalyseur comporte 0 % en poids de cuivre ou d'un composé de cuivre dans la masse active, de préférence jusqu'à 50 % en poids, de manière davantage préférée 1 à 30 % en poids, notamment 10 à 25 % en poids, par rapport à la totalité de la masse catalytique active.

6. Procédé selon l'une quelconque des revendications 1 à 5, **caractérisé en ce que** le catalyseur est placé sur un support approprié, de préférence un support à base de charbon actif, de carbure de silicium, d'oxydes métalliques ou leurs mélanges, de manière davantage préférée d'oxyde d'aluminium, de dioxyde de silicium, de dioxyde de titane, de dioxyde de zirconium, d'oxyde de zinc, d'oxyde de magnésium ou leurs mélanges, notamment d'oxyde d'aluminium γ, de dioxyde de silicium, de dioxyde de zirconium, de dioxyde de titane ou leurs mélanges, le support représentant de préférence 10 à 90 % en poids de la masse totale du catalyseur.

7. Procédé selon l'une quelconque des revendications 1 à 6, **caractérisé en ce que** le procédé est réalisé à des pressions de 5 à 350 bars, de préférence de 5 à 200 bars, de manière particulièrement préférée de 10 à 100 bars, notamment de 10 à 30 bars, et à des températures de 50 à 250 °C, de préférence de 90 à 170 °C, notamment de 120 à 160 °C.

8. Procédé selon l'une quelconque des revendications 1 à 7, **caractérisé en ce que** le procédé est réalisé avec une charge catalytique de 0,1 à 2 kg·l⁻¹·h⁻¹, de préférence de 0,8 à 1,2 kg·l⁻¹·h⁻¹, par rapport à l'amine primaire.

9. Procédé selon l'une quelconque des revendications 1 à 8, **caractérisé en ce que** le procédé est réalisé de manière discontinue ou de manière continue, de préférence de manière continue, notamment dans un réacteur tubulaire.
